# EUROPEAN PATENT SPECIFICATION

(11) **EP 4 019 025 B1**
(45) Date of publication and mention of the grant of the patent: **06.05.2026**
(21) Application number: 20856933.5
(22) Date of filing: 21.08.2020
(51) Int. Cl.: A61K 35/12, A61K 9/50, A61K 35/28, A61K 35/39, A61K 47/36, A61K 47/42, A61P 3/10, A61K 47/34

(54) **COMPOSITION COMPRISING MICROCAPSULE AND CELL STRUCTURE**
ZUSAMMENSETZUNG MIT MIKROKAPSEL UND ZELLSTRUKTUR
COMPOSITION COMPRENANT UNE MICROCAPSULE ET UNE STRUCTURE CELLULAIRE

(30) Priority: 23.08.2019 JP 2019152510
(43) Date of publication of application: 29.06.2022
(73) Proprietor: FUJIFILM Corporation, Tokyo 106-8620 (JP)
(72) Inventor: NAKAMURA Kentaro, Ashigarakami-gun, Kanagawa 258-8577 (JP); KOGAWA Ryo, Ashigarakami-gun, Kanagawa 258-8577 (JP); TAKEGAMI Ryuta, Ashigarakami-gun, Kanagawa 258-8577 (JP); MOCHIZUKI Yusuke, Ashigarakami-gun, Kanagawa 258-8577 (JP)
(74) Representative: Hoffmann Eitle
(86) International application number: PCT/JP2020/031546
(87) International publication number: WO 2021/039610

(56) References cited:
- WO-A1-2016/052504
- WO-A1-2019/044990
- WO-A1-2019/044990
- DARRABIE ET AL: "Characteristics of Poly-l-Ornithine-coated alginate microcapsules", BIOMATERIALS, ELSEVIER, AMSTERDAM, NL, vol. 26, no. 34, 1 December 2005 (2005-12-01), pages 6846 - 6852, XP005000775, ISSN: 0142-9612, DOI: 10.1016/J.BIOMATERIALS.2005.05.009
- ITO T ET AL: "Mesenchymal Stem Cell and Islet Co-Transplantation Promotes Graft Revascularization and Function", TRANSPLANTATION, WILLIAMS AND WILKINS, GB, vol. 89, no. 12, 27 June 2010 (2010-06-27), pages 1438 - 1445, XP002722620, ISSN: 0041-1337, DOI: 10.1097/TP.0B013E3181DB09C4
- KERBY, A. ET AL.: "Co-transplantation of islets with mesenchymal stem cells in microcapsules demonstrates graft outcome can be improved in an isolated- graft model of islet transplantation in mice", CYTOTHERAPY, vol. 15, no. 2, 2013, pages 192 - 200, XP055091463, ISSN: 1465-3249, DOI: 10.1016/j.jcyt. 2012.10.01 8
- WILLIAM F. KENDALLJR., EMMANUEL C. OPARA: "Polymeric Materials for Perm-Selective Coating of Alginate Microbeads", CELL MICROENCAPSULATION , vol. 1479, 2017, pages 95 - 109, XP009533801, ISSN: 1064-3745, ISBN: 978-1-4939-6362-1, DOI: 10.1007/978-1-4939-6364-5 7
- ANDO YUSUKE: "Current status of clinical trials about the bioartificial pancreas", ORGAN BIOLOGY, vol. 24, no. 1, 2017, pages 7 - 12, XP055797786, ISSN: 1340-5152

## Description

### Technical Field

The present invention relates to a composition containing at least microcapsules, cell structures and one or more selected from an insulin-secreting cell, a pancreatic islet cell, and a pancreatic islet, in which at least a part of the cell structures and at least a part of the one or more selected from an insulin-secreting cell, a pancreatic islet cell, or a pancreatic islet are encapsulated in the microcapsules.

### Related Art

There is a method called pancreatic islet transplantation as an advanced treatment method that can be a radical treatment for diabetes. The pancreatic islet transplantation is an advanced treatment method that eliminates the need for insulin injection in diabetes for each meal, and specifically, it is a treatment method of transplanting pancreatic islets (insulin-secreting tissue) isolated from the pancreas of the organ donor. However, it is said that the pancreatic islet transplantation is difficult to become general medical treatment due to the practical problem of a shortage of organ donors. As a means to solve this problem, attempts to transplant pancreatic islets derived from heterologous animals (mostly derived from pigs), or attempts to produce pancreatic islet cells from embryonic stem cells (ES cells) or induced pluripotent stem cells (iPS cells) have been studied. Heterogeneous pancreatic islets are considered to be a source of pancreatic islets closest to industrialization.

Patent Document 1 discloses a method of treating diabetes by using neopancreatic islets containing (a) dedifferentiated pancreatic islet cell and mesenchymal stem cell and/or an adipose stem cell, or (b) redifferentiated mesenchymal stem cell which is treated to promote redifferentiation of a cell and/or an adipose stem cell. In addition, immunoisolation of a cell with an alginate capsule is described in Non-Patent Document 1. Non-Patent Document 2 describes alginate microencapsulated islets and the provision of a permeation barrier between the graft tissue and the host immune system.

### Prior Art Documents

### Patent Documents

Patent Document 1: JP2018-530603A

### Non-Patent Documents

Non-Patent Document 1: Stephan, S et al. (2005) Diabetes. 54 (3): 687-693
Non-Patent Document 2: Darrabie, M. D. et al. (2005) Biomaterials 26 :6846-6852

### SUMMARY OF THE INVENTION

There are two problems in applying heterologous pancreatic islets to treatment.

The first problem is the "avoidance of immune rejection". This is a problem in that the administered heterologous pancreatic islets are eliminated by the recipient's immune system. The use of immunosuppressive agents can be considered as a solution to the above problem; however, it cannot be said to be a practical solution since it involves an economic burden due to the permanent use of expensive immunosuppressive agents and a physical burden due to side effects. Regarding the avoidance of immune rejection, attempts to immunoisolate a cell with an alginate capsule have been studied, for example, as in Stephan, S et al. (2005) Diabetes. 54 (3): 687-693. However, as a practical problem, it is recognized as a problem that pancreatic islets cannot continue to exhibit their sustainable functions only by immunoisolation with a capsule or the like.

The second problem is the "avoidance of poor engraftment of pancreatic islets". From the viewpoint of convenience, simple subcutaneous administration is urgently desired in actual medical treatment; however, it has been known that the engraftment rate of pancreatic islets is remarkably low in the subcutaneous tissue, and thus it has been difficult to realize subcutaneous transplantation. In addition, this problem of poor engraftment of pancreatic islets becomes more apparent in pancreatic islets immunoisolated with such a capsule described above. A capsule or the like, which is used in the immunoisolation technique, has a function similar to a semipermeable membrane, which allows insulin to permeate while blocking the host's immune response to the pancreatic islets; however, in reality, such performance similar to a semipermeable membrane causes permeation inhibition of nutrients, and it has been well known that pancreatic islets inside the capsule undergo necrosis or undergo cell death through apoptosis.

An object to be achieved by the present invention is to provide a composition containing at least microcapsules and cell structures, where the composition can exhibit a high treatment effect in a case of being transplanted into a living body.

As a result of diligent studies to achieve the above object, the inventors of the present invention have found that in a case where a composition contains microcapsules containing a polymer hydrogel and contains cell structures containing a biocompatible polymer block and a mesenchymal stem cell, and in the composition, in a case where the cell structures are encapsulated in the microcapsules, heterologous cells can be engrafted and a treatment effect can be exhibited. The present invention has been completed based on the above findings.

That is, according to the present invention, the following inventions are provided.
<1> A composition comprising at least; (a) a microcapsule containing a polymer hydrogel,
   (b) a cell structure containing a biocompatible polymer block and a cell, in which a plurality of biocompatible polymer blocks are arranged in gaps between a plurality of cells, and
   (c) one or more selected from an insulin-secreting cell, a pancreatic islet cell, and a pancreatic islet,
   where at least a part of the cell structures and at least a part of the one or more selected from an insulin-secreting cell, a pancreatic islet cell, or a pancreatic islet are encapsulated in the microcapsules,
   the cell is a somatic stem cell, and
   the biocompatible polymer blocks are constituted by a polymer composed of at least one biodegradable material selected from the group consisting of a polypeptide, a polylactic acid, a polyglycolic acid, polylactic acid-co-glycolic acid (PLGA), hyaluronic acid, glycosaminoglycan, proteoglycan, chondroitin, cellulose, agarose, carboxymethyl cellulose, chitin, and chitosan.
<2> The composition according to <1>, in which the cell is a mesenchymal stem cell.
<3> The composition according to <1> or <2>, in which at least a part of the cell structures are present outside the microcapsules.
<4> The composition according to any one of <1> to <3>, in which 50% or more of the cell structures are present inside the microcapsules.
<5> The composition according to any one of <1> to <4>, in which 50% or more of the (c) one or more selected from an insulin-secreting cell, a pancreatic islet cell, or a pancreatic islet are encapsulated in the microcapsules.
<6> The composition according to any one of <1> to <5>, in which the composition contains a pancreatic islet, and a circle-equivalent diameter size of the cell structure is 0.5 times or more and 4 times or less with respect to the pancreatic islet.
<7> The composition according to any one of <1> to <6>, in which the composition contains a pancreatic islet, and a number ratio of the cell structures to the pancreatic islets, which are encapsulated in one microcapsule, is in a range of 0.1:1.0 to 1.0:0.1.
<8> The composition according to any one of <1> to <7>, in which a main component of the microcapsule is alginic acid.
<9> The composition according to any one of <1> to <8>, in which the microcapsule is coated with poly-L-ornithine.
<10> The composition according to any one of <1> to <9>, in which the composition is used for transplantation into a living body.
<11> The composition according to any one of <1> to <10>, in which the composition is for intraperitoneal transplantation or subcutaneous transplantation.
<12> The composition according to <11>, in which the composition is for subcutaneous transplantation.
<13> The composition according to any one of <1> to <12>,
   in which the composition is a pharmaceutical composition.
<14> The composition according to any one of <1> to <13>, in which the composition is a pharmaceutical composition for treatment of diabetes.

The scope of the invention is defined in the appended claims. **In** addition, the present disclosure describes the following items <A> to <C>.
<A> A method of transplanting, into a living body, a composition comprising at least, (a) a microcapsule containing a polymer hydrogel, and
   (b) a cell structure containing a biocompatible polymer block and a cell, in which a plurality of biocompatible polymer blocks are arranged in gaps between a plurality of cells,
   where in the composition, at least a part of the cell structures are encapsulated in the microcapsules.
<B> A method of treating diabetes by transplanting, into a living body, a composition comprising at least, (a) a microcapsule containing a polymer hydrogel, and
   (b) a cell structure containing a biocompatible polymer block and a cell, in which a plurality of biocompatible polymer blocks are arranged in gaps between a plurality of cells,
   where in the composition, at least a part of the cell structures are encapsulated in the microcapsules.
<C> Use of a composition, for producing of a pharmaceutical composition for treatment of diabetes, the pharmaceutical composition comprising at least,
   (a) a microcapsule containing a polymer hydrogel, and
   (b) a cell structure containing a biocompatible polymer block and a cell, in which a plurality of biocompatible polymer blocks are arranged in gaps between a plurality of cells,
   where at least a part of the cell structures are encapsulated in the microcapsules.

According to the composition according to an aspect of the present invention, a high treatment effect can be exhibited in a case of being transplanted into a living body.

### BRIEF DESCRIPTION OF THE DRAWINGS

Fig. 1 is a graph showing transition of a blood glucose level (a median value) in a case of intraperitoneal transplantation has been carried out.
Fig. 2 is a graph showing transition of a blood glucose level (a median value) in a case of subcutaneous transplantation has been carried out.
Fig. 3 is a graph showing transition of a control rate of blood glucose level in a case of intraperitoneal transplantation has been carried out.
Fig. 4 is a graph showing transition of a control rate of blood glucose level in a case of subcutaneous transplantation has been carried out.
Fig. 5 is an image showing sodium alginate microcapsule that encapsulates pancreatic islets and cell structures.

### DESCRIPTION OF THE PREFERRED EMBODIMENTS

Hereinafter, the embodiments of the present invention will be described in detail.

The composition according to the embodiment of the present invention is a composition containing at least;
(a) a microcapsule containing a polymer hydrogel,
(b) a cell structure containing a biocompatible polymer block and a cell, in which a plurality of biocompatible polymer blocks are arranged in gaps between a plurality of cells, and
(c) one or more selected from an insulin-secreting cell, a pancreatic islet cell, and a pancreatic islet,

where at least a part of the cell structures and at least a part of the one or more selected from an insulin-secreting cell, a pancreatic islet cell, or a pancreatic islet are encapsulated in the microcapsules,
the cell is a somatic stem cell, and
the biocompatible polymer blocks are constituted by a polymer composed of at least one biodegradable material selected from the group consisting of a polypeptide, a polylactic acid, a polyglycolic acid, polylactic acid-co-glycolic acid (PLGA), hyaluronic acid, glycosaminoglycan, proteoglycan, chondroitin, cellulose, agarose, carboxymethyl cellulose, chitin, and chitosan.

### <Microcapsule>

It is sufficient that the microcapsule containing a polymer hydrogel is a spherical or substantially spherical particle.

The microcapsule is usually a particle having a size equivalent to that of a sphere having a diameter of 100 µm to 8,000 µm, preferably a diameter of 200 µm to 5,000 µm, and more preferably a diameter of 300 µm to 3,000 µm; however, the size thereof is not particularly limited. That is, the "micro" of the microcapsule has no particular meaning for limiting the size.

The polymer hydrogel refers to a gel that forms a three-dimensional network structure by the crosslinking of a polymer and is swelled by absorbing a solvent in the inside thereof, and it is a substance that has intermediate properties between solid and liquid.

The microcapsule preferably has an immunoisolation function. Immunoisolation is a method for preventing immune rejection. In general, the immunoisolation is one of the methods for preventing the recipient's immune rejection at the time of transplantation. Here, the immune rejection is the recipient's rejection of the cells that are transplanted to the recipient. The immunoisolation isolates cells from the recipient's immune rejection. Examples of the immune rejection include the immune rejection due to a cell-mediated immune response and the immune rejection due to a humoral immune response.

The microcapsule preferably contains a biocompatible polymer. Specifically, the microcapsule preferably contains a polymer hydrogel formed by crosslinking a biocompatible polymer. Biocompatibility can also be represented as biocompatibility.

Regarding the biocompatible polymers, JP2018-521717A (WO2016/187225A), US5709854A, US6129761A, and US6858229B can be referenced.

Specific examples of the polymer hydrogel include polysaccharide, collagen, sodium cellulose sulfate, gelatin, a water-soluble polyacrylate, polyphosphazine, polyvinylpyrrolidone (PVP), polyethylene glycol, polyacrylic acid, poly(methacrylic acid), poly(alkylene oxide), 2-methacryloyloxyethyl phosphorylcholine (MPC), and alginic acid, chitosan, agarose, hyaluronic acid, or gelatin is preferable, and alginic acid is more preferable. Further, a mixture of any two or more of them can be used.

Examples of the polysaccharide include alginic acid, chitosan, hyaluronic acid (also referred to as hyaluronan), chondroitin sulfate, and agarose.

In a case where the conditions for forming solutions of alginic acid, chitosan, and agarose, or the compositions of these solutions are adjusted, these solutions can be made into a cross-linked hydrogel. On the other hand, in a case of being modified to contain a crosslinkable group, hyaluronan and chondroitin sulfate can typically be made into a hydrogel.

The main component of the microcapsule is preferably alginic acid.

Alginic acid is a block copolymer composed of glucuronic acid (G) and mannuronic acid (M). Alginic acid is naturally present as a cell wall-constituting polysaccharide or an intercellular filling substance of brown algae and can be collected using these as a raw material. Specific examples of the raw material brown algae include brown algae of the order Fucales, family Durvillaeaceae, genus Durvillaea (for example, D. potatorum), the order Fucales, family Fucaceae, genus Ascophyllum (for example, A. nodosum), the order Laminariales, family Laminariaceae, genus Saccharina (for example, Saccharina japonica and Saccharina longissima), the order Laminariales, family Lessoniaceae, genus Eisenia (for example, Eisenia bicyclis), the order Laminariales, family Lessoniaceae, genus Ecklonia (for example, Ecklonia cava and Kcklonia Kurome), and the order Laminariales, family Lessoniaceae, genus Lessonia (for example, L. flavikans). Alternatively, commercially available alginic acid can be used.

Alginic acid has a structure in which two kinds of uronic acids, mannuronic acid (M) and guluronic acid (G), are linearly polymerized. The ratio of guluronic acid to mannuronic acid is generally represented as an M/G ratio. The M/G ratio of the alginic acid is not particularly limited. However, the smaller the M/G ratio is, the better the gel hardness is and the greater the gel-forming ability is, and thus the M/G ratio is preferably small. Specifically, it is preferably 0.2 to 2.0 and more preferably 0.5 to 1.25. On the other hand, the molecule permeation rate in the gel is faster in a case where the M/G ratio is larger, and thus there is an optimum range of the M/G ratio.

It is presumed that a polyvalent metal cation invades the pocket structure included in the M block to form an egg box, whereby alginic acid is gelated. In the present invention, it is sufficient that the microcapsule is formed by utilizing this gelation. Specific examples of the polyvalent metal cation that can cause gelation of alginic acid include a divalent or trivalent ion of a metal such as calcium (Ca), barium (Ba), aluminum (Al), magnesium (Mg), copper (Cu), strontium (Sr), cadmium (Cd), zinc (Zn), nickel (Ni), cobalt (Co), manganese (Mn), iron (Fe), or tin (Sn). Among the above, a calcium ion, a magnesium ion, a barium ion, or a strontium ion is preferable, and a calcium ion is more preferable.

That is, alginic acid may be in the form of alginate in the microcapsule. In addition, alginate may be used as a raw material of the alginic acid, with which the microcapsule is formed. Here, preferred examples of the alginate include sodium alginate.

Further, alginic acid may be an alginic acid derivative. Regarding the alginic acid derivative, US9422373B can be referred to.

The molecular weight of alginic acid is preferably 50 kDa or more, more preferably 100 kDa or more, and still more preferably 150 kDa or more. The membrane of the sodium alginate microcapsule that is used in Examples which will be described later can block substances of 500 kDa or more for 24 hours or more and can block substances of 150 kDa or more for 2 hours or more.

In the microcapsule, alginic acid preferably further forms a polyion complex gel together with a polycation (an organic polymer compound having a cation residue). For example, in a case where a hydrogel formed by ionically cross-linking alginic acid with a divalent cation further comes into contact with a polycation, a semi-permeable membrane complexed with alginic acid mainly composed of a polycation can be formed. At this time, in general, a microcapsule that has an inner shell consisting of an alginic acid gel and an outer shell containing a polycation is formed. This microcapsule may further have an additional outermost layer shell (for example, an envelope). For example, in a case where an additional outermost layer shell is formed of an alginic acid gel, the surface charge can be reduced. Specifically, a multi-layer microcapsule of "an alginic acid gel / a polycation-alginic acid gel / an alginic acid gel / insulin-secreting cells" may be formed.

Here, the polyion complex state refers to a state in which an organic polymer compound having a cation residue and an organic polymer compound having an anion residue form a composite body by electrostatic interaction, and the microcapsule is in a state of being coated with a polycation.

Examples of the polycation include a polymer having a basic reactive group such as an amine group or an imine group. Specific examples thereof include polyornithine (poly(L-ornithine)), polylysine (poly(L-lysine)), chitosan, gelatin, collagen, polyethyleneimine, poly(vinylamine), and poly(allylamine), polyornithine or polylysine is preferable, and polyornithine is more preferable. That is, it is preferable that the microcapsule is coated with poly-L-ornithine. The molecular weight of the polycation is preferably 1,500 to 300,000 and more preferably 3,000 to 150,000.

Regarding the alginic acid hydrogel crosslinked with a polycation, Journal of biomedical materials research Part B 101B 258-268 (2013) can be referenced.

### <Cell structure>

In the present specification, the cell structure means a structure including a biocompatible polymer block and a cell, and a plurality of the polymer blocks are arranged in gaps between the plurality of the cells. The structure for transplantation according to the embodiment of the present invention contains a biocompatible polymer block and a mesenchymal stem cell, and it preferably contains a cell structure in which a plurality of the polymer blocks are arranged in the gap between the plurality of the mesenchymal stem cells.

In the cell structure, in a case where a plurality of polymer blocks are three dimensionally arranged in the mosaic pattern in the gaps between a plurality of cells by using a biocompatible polymer block and a cell, the polymer block having biocompatibility and the cells are three dimensionally arranged in the mosaic pattern, and thus a cell three-dimensional structure in which the cells are uniformly present in the structure is formed.

In the cell structure, the plurality of polymer blocks are arranged in a gap between the plurality of cells. Here, the "gap between cells" is not necessarily a space closed by the constituent cells and may be interposed by the cells. Gaps are not necessarily present between all the cells, and there may be a place where cells are brought into contact with each other. The distance of the gap between cells through polymer blocks, that is, the gap distance in a case of selecting a certain cell, and a cell existing in the shortest distance from the certain cell is not particularly limited. However, the distance is preferably the size of a polymer block, and an appropriate distance is also within a range of the appropriate size of a polymer block.

The polymer block refers to a block containing a polymer. It may be spherical, porous, flat, granular, or amorphous, and is preferably amorphous. In addition, the polymer block has a configuration of being interposed by the cells. However, there are not necessarily cells between all of the polymer blocks, and there may be a place where polymer blocks are brought into contact with each other. The distance between the polymer blocks through cells, that is, the distance between the polymer blocks in a case of selecting a polymer block, and a polymer block existing in the shortest distance from the polymer block is not particularly limited. However, the distance therebetween is preferably the size of an aggregation of cells in a case where one or several cells to be used are gathered. For example, the size thereof is 10 µm or more and 1,000 µm or less, preferably 10 µm or more and 100 µm or less, and more preferably 10 µm or more and 50 µm or less.

The polymer constituting the biocompatible polymer block is composed of a biodegradable material, which is at least one material selected from the group consisting of a polypeptide, a polylactic acid, a polyglycolic acid, polylactic acid-co-glycolic acid (PLGA), hyaluronic acid, glycosaminoglycan, proteoglycan, chondroitin, cellulose, agarose, carboxymethyl cellulose, chitin, and chitosan. Among the above, a polypeptide is particularly preferable. The kind of the polypeptide is not particularly limited as long as the polypeptide has biocompatibility; however, for example, gelatin, collagen, elastin, fibronectin, ProNectin, laminin, tenascin, fibrin, fibroin, entactin, thrombospondin, or RetroNectin is preferable, and gelatin, collagen, or atelocollagen is most preferable. As the gelatin to be used in the present invention, a natural gelatin or a recombinant gelatin is preferable. A recombinant gelatin is more preferable.

The recombinant gelatin means polypeptides or protein-like substances which have an amino acid sequence similar to that of gelatin produced through gene recombination technology. The recombinant gelatin which can be used in the present invention preferably has a repetition of a sequence (X and Y each independently represent any amino acids) represented by Gly-X-Y which is characteristic of collagen. Here, a plurality of pieces of Gly-X-Y may be the same as or different from each other. Preferably, two or more sequences of cell adhesion signals are included in one molecule. As the recombinant gelatin that is used in the present invention, gelatin having an amino acid sequence derived from the partial amino acid sequence of collagen can be used. For example, it is possible to use those disclosed in EP1014176B, US6992172B, WO2004/85473A, WO2008/103041A, and the like. However, the recombinant gelatin is not limited thereto. The recombinant gelatin that is used in the present invention is preferably gelatin having the following aspects.

The recombinant gelatin is excellent in biocompatibility with original performance of natural gelatin, and is excellent in non-infection properties since there is no concern of bovine spongiform encephalopathy (BSE) and the recombinant gelatin with not being naturally derived. In addition, the recombinant gelatin is uniform as compared with natural gelatin, and the sequence thereof is determined. As a result, it is possible to reduce deviation by crosslinking or the like and to carry out design accurately in terms of strength and decomposition.

The molecular weight of recombinant gelatin is not particularly limited; however, it is preferably 2,000 or more and 100,000 or less (2 kDa or more and 100 kDa or less), more preferably 2,500 or more and 95,000 or less (2.5 kDa or more and 95 kDa or less), still more preferably 5,000 or more and 90,000 or less (5 kDa or more and 90 kDa or less), and most preferably 10,000 or more and 90,000 or less (10 kDa or more and 90 kDa or less).

The molecular weight distribution of the recombinant gelatin is not particularly limited; however, it is preferable that in the molecular weight distribution measurement, recombinant gelatin has an area rate of the maximum molecular weight peak of 70% or more, more preferably 90% or more, and most preferably 95% or more, with respect to the total area of all the molecular weight peaks. The molecular weight distribution of recombinant gelatin can be measured by the method disclosed in PCT/JP2017/012284A.

The recombinant gelatin preferably has a repetition of a sequence represented by Gly-X-Y which is characteristic of collagen. Here, a plurality of pieces of Gly-X-Y may be the same as or different from each other. In Gly-X-Y, Gly represents glycine, and X and Y represent any amino acid (preferably represents any amino acid other than glycine). The sequence represented by Gly-X-Y characteristic to collagen is a partial structure which is extremely specific compared to other proteins in a composition or a sequence of an amino acid of gelatin and collagen. In this section, glycine occupies about one third of the entirety of the amino acid sequence, and one sequence is repeated every three sequences. Glycine is the simplest amino acid. Therefore, there is a little restraint in arrangement of molecular chains and glycine significantly and contributes to regeneration of a helix structure during gelation. It is preferable that amino acids represented by X and Y contain many imino acids (proline and oxyproline) and occupy 10% to 45% of the entirety of the sequence. Preferably 80% or more of the sequence of the amino acids, more preferably 95% or more of the sequence of the amino acids, and most preferably 99% or more of the sequence of the amino acids in the recombinant gelatin has a repeating structure of Gly-X-Y.

In the polar amino acids of the general gelatin, charged ones and non-charged ones exist by 1:1. Here, the polar amino acid specifically indicates cysteine, aspartic acid, glutamic acid, histidine, lysine, asparagine, glutamine, serine, threonine, tyrosine, and arginine. Among these, the polar non-charged amino acid indicates cysteine, asparagine, glutamine, serine, threonine, and tyrosine. In the gelatin that is used in the present invention, the proportion of polar amino acids to all the constituent amino acids is 10% to 40% and preferably 20% to 30%. It is preferable that the proportion of a non-charged amino acid in the polar amino acid is equal to or more than 5% and less than 20% and preferably equal to or more than 5% and less than 10%. Furthermore, it is preferable that any one amino acid or preferably two or more amino acids among serine, threonine, asparagine, tyrosine, and cysteine are not contained in the sequence.

In general, in polypeptides, minimum amino acid sequences which work as cell adhesion signals are known (for example, "Pathophysiology", Vol. 9, No. 7 (1990) p. 527 published by Nagai Shoten Co., Ltd.). The gelatin that is used in the present invention preferably has two or more of these cell adhesion signals in one molecule. As the specific sequences, sequences such as an RGD sequence, an LDV sequence, an REDV sequence, a YIGSR sequence, a PDSGR sequence, an RYVVLPR sequence, an LGTIPG sequence, an RNIAEIIKDI sequence, an IKVAV sequence, an LRE sequence, a DGEA sequence, and a HAV sequence, which are represented by one-letter notation of amino acids are preferable in that there are many kinds of cells adhered. An RGD sequence, a YIGSR sequence, a PDSGR sequence, an LGTIPG sequence, an IKVAV sequence, and a HAV sequence are more preferable and an RGD sequence is particularly preferable. In the RGD sequence, an ERGD sequence is preferable. In a case where gelatin having a cell adhesion signal is used, the amount of substrate produced by cells can be increased. For example, in a case of cartilage differentiation using a mesenchymal stem cell as a cell, the production of glycosaminoglycan (GAG) can be increased.

Regarding the arrangement of RGD sequences in the recombinant gelatin, it is preferable that the number of amino acids between RGDs is not uniform between 0 and 100 and preferably between 25 and 60.

From the viewpoint of cell adhesion and proliferation, the content of the above minimum amino acid sequence is preferably 3 to 50 sequences, more preferably 4 to 30 sequences, and particularly preferably 5 to 20 sequences in one protein molecule. 12 sequences are most preferable.

In recombinant gelatin, the proportion of RGD motifs with respect to the total number of amino acids is preferably at least 0.4%. In a case where recombinant gelatin contains 350 or more amino acids, each stretch of the 350 amino acids preferably contains at least one RGD motif. The proportion of RGD motifs with respect to the total number of amino acids is more preferably at least 0.6%, still more preferably at least 0.8%, even still more preferably at least 1.0%, particularly preferably at least 1.2%, and most preferably at least 1.5%. The number of RGD motifs in the peptide is preferably at least 4, more preferably at least 6, still more preferably at least 8, and even still more preferably 12 or more and 16 or less per 250 amino acids. The proportion of RGD motifs being 0.4% corresponds to at least one RGD sequence per 250 amino acids. Since the number of RGD motifs is an integer, the recombinant gelatin consisting of 251 amino acids has to contain at least two RGD sequences to satisfy the feature of at least 0.4%. It is preferable that the recombinant gelatin contains at least two RGD sequences per 250 amino acids, more preferably contains at least three RGD sequences per 250 amino acids, and still more preferably contains at least four RGD sequences per 250 amino acids. Further, another aspect of the recombinant gelatin in the present invention includes at least 4 RGD motifs, preferably at least 6 RGD motifs, more preferably at least 8 RGD motifs, and still more preferably 12 or more and 16 or less RGD motifs.

In addition, the recombinant gelatin may be partially hydrolyzed.

The recombinant gelatin is preferably represented by A-[(Gly-X-Y)ₙ]ₘ-B. n pieces of X each independently represent any amino acid and n pieces of Y each independently represent any amino acid. m preferably represents an integer of 2 to 10 and more preferably represents an integer of 3 to 5. n is preferably an integer of 3 to 100, more preferably an integer of 15 to 70, and most preferably an integer of 50 to 65. A represents any amino acid or an amino acid sequence, and B represents any amino acid or an amino acid sequence. Here, n pieces of Gly-X-Y may be the same as or different from each other.

More preferably, the recombinant gelatin is represented by, Formula: Gly-Ala-Pro-[(Gly-X-Y)₆₃]₃-Gly (In the formula, 63 pieces of X each independently represent any amino acid and 63 pieces of Y each independently represent any amino acid. Here, 63 pieces of Gly-X-Y may be the same as or different from each other).

It is preferable that a plurality of sequence units of collagen which naturally exists are bonded to a repeating unit. The naturally occurring collagen referred to here may be any naturally occurring collagen as long as it is naturally present; however, it is preferably type I, type II, type III, type IV, or type V collagen. It is more preferably type I, type II, or type III collagen. According to another form, the above-described collagen is preferably derived from a human, cattle, a pig, a mouse, or a rat, and is more preferably derived from a human.

An isoelectric point of the recombinant gelatin is preferably 5 to 10, more preferably 6 to 10, and still more preferably 7 to 9.5. The measurement of the isoelectric point of recombinant gelatin can be carried out by passing a solution of 1% by mass of recombinant gelatin through a mixed crystal column of a cation and anion exchange resins and then measuring the pH according to isoelectric focusing (see Maxey, C. R. (1976); Phitogr. Gelatin 2, Editor Cox, P. J. Academic, London, Engl.).

It is preferable that the recombinant gelatin is not deaminated.

It is preferable that the recombinant gelatin does not have a telopeptide.

It is preferable that the recombinant gelatin is a substantially pure polypeptide which is prepared using a nucleic acid encoding an amino acid sequence.

It is particularly preferable that the recombinant gelatin is any of;
(1) a peptide formed of an amino acid sequence described in SEQ ID NO: 1;
(2) a peptide which is formed of an amino acid sequence in which one or several amino acids are deleted, substituted, or added in the amino acid sequence disclosed in SEQ ID NO: 1, and has biocompatibility; or
(3) a peptide consisting of an amino acid sequence which has 80% or more (more preferably 90% or more, particularly preferably 95% or more, and most preferably 98% or more) of a sequence identity with the amino acid sequence set forth in SEQ ID NO: 1, and having biocompatibility.

"One or several" in the "amino acid sequence in which one or several amino acids are deleted, substituted, or added" preferably means 1 to 20 amino acids, more preferably means 1 to 10 amino acids, still more preferably means 1 to 5 amino acids, and particularly preferably means 1 to 3 amino acids.

The recombinant gelatin can be produced through gene recombination technology which is known to those skilled in the art and can be produced in accordance with, for example, methods disclosed in EP1014176A2, US6992172B, WO2004/85473A, and WO2008/103041A. Specifically, a gene encoding an amino acid sequence of predetermined recombinant gelatin is acquired, the acquired gene is incorporated into an expression vector to produce a recombinant expression vector, and a transformant is produced by introducing the recombinant expression vector into an appropriate host. The recombinant gelatin is produced by culturing the obtained transformant in an appropriate medium. Therefore, it is possible to prepare the gelatin that is used in the present invention by collecting the recombinant gelatin produced from a culture product.

The cell structure contains a cell.

The cell is a somatic stem cell. Examples of the somatic stem cell capable of being used include a mesenchymal stem cell (MSC), a hematopoietic stem cell, an amnion cell, an umbilical cord blood cell, and a bone marrow-derived cell (for example, a bone marrow-derived MSC), a myocardial stem cell, an adipose-derived stem cell, and a neural stem cell. Among the above, a mesenchymal stem cell is preferable, an adipose-derived mesenchymal stem cell or a bone marrow-derived mesenchymal stem cell is more preferable, and an adipose-derived mesenchymal stem cell is still more preferable. The mesenchymal stem cell refers to a somatic stem cell present in the mesenchymal tissue and has an ability to differentiate into a cell belonging to the mesenchymal tissue. The mesenchymal tissue refers to tissue such as bone, cartilage, adipose, blood, bone marrow, skeletal muscle, dermis, ligament, tendon, or heart. The somatic stem cells is collected and isolated from a tissue of a living body in the classical understanding: however, it can also be created by being derived from an embryonic stem cell (an ES cell) or an induced pluripotent stem cell (an iPS cell). In the present specification, not only a cell derived from a tissue of a living body but also a cell derived from an ES cell or an iPS cell can be used.

The origin of the cell is not particularly limited as long as the cell is a mammalian cell. It is a cell derived preferably from a human, a cat, a dog, or a pig, and more preferably from a human. In addition, it may be of allogeneic origin or of xenogeneic origin. It is preferably of xenogeneic origin from the viewpoint of the easy availability of the cell. In a case of being of allogeneic origin, it may be of autologous origin or of heterogeneous origin. It is preferably of heterogeneous origin from the viewpoint of the easy availability of the cell.

The size of the cell structure is, for example, 110 to 1,000 µm or less, and it is preferably 150 to 800 µm or less and more preferably 210 µm to 600 µm or less.

As another example of the cell in the cell structure, a cell that produces a useful substance is preferable. Examples of the useful substance include a hormone (insulin or the like), a cytokine (interferon, interleukin, tumor necrosis factor, colony stimulating factor, growth factor, or the like), an enzyme (a lysosomal storage disease-related enzyme or the like), a neurotransmitter (dopamine or the like), an antibody, an antigen, and another physiologically active substance (a protein, a peptide, or the like.); however, the examples are not limited thereto. The cell that produces a useful substance may be non-transformed cells such as a skin cell, a cartilage cell, a liver cell, or a renal cell, or may be a transformed cell into which a gene encoding a useful substance or a gene involved in the biosynthesis of a useful substance is introduced. Further, the cells may be cells derived from a living body or cells derived from an embryonic stem cell or an induced pluripotent stem cell.

Details and preferred embodiments of the cell structure are described in WO2011/108517A, JP2014-12114A, WO2014/133081A, JP2015-134193A, and WO2015/190430A.

At least a part of the cell structures are encapsulated in the microcapsules. At least a part of the cell structures may be present outside the microcapsule. That is, examples of the aspect of the composition according to the embodiment of the present invention include an aspect in which all of the cell structures are encapsulated in the microcapsules and an aspect in which a part of the cell structures are encapsulated in the microcapsules and a part of the cell structures are present outside the microcapsules. 50% or more of the cell structures are preferably encapsulated in microcapsules, 80% or more thereof are more preferably encapsulated in the microcapsules, and 90% or more thereof are particularly preferably encapsulated therein.

### <Insulin-secreting cell, pancreatic islet cell, and pancreatic islet>

The composition according to the embodiment of the present invention further contains the (c) one or more selected from an insulin-secreting cell, a pancreatic islet cell, and a pancreatic islet.

The insulin-secreting cell refers to a cell that can secrete insulin in response to the change in blood glucose level.

Insulin is a polypeptide (having a molecular weight of about 6,000) in which an A chain of 21 amino acid residues and a B chain of 30 amino acid residues are linked through a disulfide bond. In the living body of a mammal, insulin is secreted from β cells present in the pancreatic islet (the islet of Langerhans in the pancreas). The insulin may be human insulin or other mammalian (for example, porcine) insulin. The insulin may be insulin produced by a genetic recombination method. Regarding the method of obtaining genetically recombinated insulin, for example, the description in Diabetes Navigator; edited by Takashi Kadowaki (pages 270 to 271, Ken Tao, Yoshikazu Oka "Current and Future Insulin Pharmaceutical Preparations", Medical Review Co., Ltd., 2002) can be referenced. Various insulin analogs (see, for example, H. C. Lee, J. W. Yoon, et al., Nature, Vol. 408, pp. 483-488, 2000) may be used.

The insulin-secreting cells are not particularly limited, and examples thereof include pancreatic β cells present in the islet of Langerhans in the pancreas. The pancreatic β cells may be human pancreatic β cells or may be porcine, murine, or other pancreatic β cells. Regarding the method of extracting pancreatic β cells from a pig, the description in JP2007-195573A can be referenced. In addition, the insulin-secreting cells may be cells induced from human stem cells (see, for example, Junichi Miyazaki, Regenerative medicine, Vol. 1, No. 2, pp. 57-61, 2002) or cells induced from small intestinal epithelial stem cells (see, for example, Mineko Fujimiya et al., Regenerative medicine, Vol. 1, No. 2, pp. 63-68, 2002), or may be insulin-secretary cells into which a gene encoding insulin is incorporated (see, for example, H. C. Lee, J. W. Yoon, et al., Nature, Vol. 408, pp. 483-488, 2000). The composition according to the embodiment of the present invention may contain pancreatic islet cells that include pancreatic β cells.

The insulin-secreting cells may be a cell aggregate. In the present specification, the cell aggregate is a cell aggregate in which a plurality of cells are aggregated. An example of the cell aggregate is a pancreatic islet. That is, the composition according to the embodiment of the present invention may contain pancreatic islets (see, for example, Hiroshi Hori, Kazutomo Inoue, Regenerative medicine, Vol. 1, No. 2, pp. 69-77, 2002).

In the composition according to the embodiment of the present invention, at least a part of the above-described (c) one or more selected from an insulin-secreting cell, a pancreatic islet cell, and a pancreatic islet are encapsulated in the microcapsules. In addition, at least a part of the above-described (c) one or more selected from an insulin-secreting cell, a pancreatic islet cell, and a pancreatic islet may be present outside the microcapsules. In at least a part of the one or more selected from an insulin-secreting cell, a pancreatic islet cell, and a pancreatic islet, 50% or more thereof is preferably present inside the microcapsules, 80% or more thereof is preferably present inside the microcapsules, and 90% or more thereof is particularly preferably present inside the microcapsules.

In a case where the composition according to the embodiment of the present invention contains pancreatic islets, the circle-equivalent diameter size of the cell structure is preferably 0.1 times or more and 10 times or less, more preferably 0.2 times or more and 5 times or less, still more preferably 0.5 times or more and 2 times or less, and particularly preferably 0.7 times or more and 1.5 times or less, with respect to the pancreatic islet. It is noted that the circle-equivalent diameter size is obtained by measuring the area of the pancreatic islet or the cell structure from the two-dimensional image of the pancreatic islet or the cell structure, dividing the area by the number π, and then doubling the square root of the obtained value.

The pancreatic islet is an aggregate containing about 2,000 cells on average. In the cell structure, the number of cells per pancreatic islet is preferably 100 to 10,000 and more preferably 600 to 2,400.

In a case where the composition according to the embodiment of the present invention contains pancreatic islets, the number ratio of cell structures to pancreatic islets, which are encapsulated in one microcapsule, is preferably in a range of 0.1:1.0 to 1.0:0.1, more preferably in a range of 0.2:1.0 to 1.0:0.2, and still more preferably in a range of 0.5:1.0 to 1.0:0.5.

### <Composition and production thereof>

The composition according to the embodiment of the present invention can be used for transplanting a transplant cell into a recipient.

In addition to the (a) a microcapsule and the (b) a cell structure, the composition according to the embodiment of the present invention may include a protein such as albumin, a peptide, a pH buffer, an inorganic salt, and the like as long as they do not interfere with the formation and the maintenance of the microcapsule.

In addition, the composition according to the embodiment of the present invention contains the (c) one or more selected from an insulin-secreting cell, a pancreatic islet cell, and a pancreatic islet, in addition to the cell that constitutes the cell structure, and in addition to the above, it may further contain another cell that supports the function of the above cell.

In order to produce the composition according to the embodiment of the present invention, first, the (b) a cell structure and the (c) one or more selected from an insulin-secreting cell, a pancreatic islet cell, and a pancreatic islet are mixed and suspend in a solution of a polymer that constitutes the microcapsule. In a case where the entire suspension is suspended well (for example, by repeatedly transferring the entire suspension between two syringes), the (b) a cell structure is homogeneously dispersed. Next, a microcapsule in which the cell structure is embedded in the polymer can be obtained from the obtained suspension by using an encapsulation machine.

For example, a cell structure suspended in a sodium alginate solution can be dropwise added into a solution containing CaCl₂ using an encapsulation machine, and alginic acid can be crosslinked to be gelated, whereby a capsule in which the cell structure is embedded can be formed. Further, it is preferable that the formed capsule is transferred to a liquid containing a polycation to coat the surface thereof.

The composition according to the embodiment of the present invention may contain an administration solution for transplantation into a living body. Examples of the administration solution include a 3 mmol/L glucose-containing medium (Cosmo Bio Co., Ltd.: PNIM4), a Hanks' balanced salt solution (HBSS), phosphate buffered saline (PBS), an ET-KYOTO solution, a University of Wisconsin (UW) solution, a Roswell Park Memorial Institute (RPMI) 1640 medium, a Connaught Medical Research Laboratories (CMRL) medium, a Dulbecco's modified Eagle medium (DMEM), physiological saline, and an isotonic solution, which are not particularly limited.

### <Use application of composition>

The composition according to the embodiment of the present invention can be used for transplantation into a living body. The composition according to the embodiment of the present invention can be transplanted, for example, intraperitoneally, subcutaneously, subrenally, or into the peritoneum, and it is preferably transplanted intraperitoneally or subcutaneously. The composition according to the embodiment of the present invention may be administered intravascularly or may be a vascular connection device. For example, in a case where the composition according to the embodiment of the present invention containing insulin-secreting cells is transplanted so that the blood comes into direct contact with the composition according to the embodiment of the present invention, the insulin secretion in response to a change in blood glucose level becomes possible.

The composition according to the embodiment of the present invention is preferably a pharmaceutical composition.

The composition according to the embodiment of the present invention can be used, for example, for the treatment of a disease that requires the transplantation of insulin-secreting cells. Examples of the disease that requires the transplantation of insulin-secreting cells include diabetes. The composition according to the embodiment of the present invention is, for example, a pharmaceutical composition for the treatment of diabetes. The diabetes includes type 2 diabetes and type 1 diabetes. In particular, the above is a great need in type 1 diabetes, and it is conceived that the effect of transplanting insulin-secreting cells and the improvement of quality of life (QOL) are high.

The recipient to be subjected to transplantation is preferably a mammal and more preferably a human.

The number of transplantations of the structure for transplantation according to the embodiment of the present invention may be one time and may be two or more times as necessary.

In a case of using insulin-secreting cells, a donor and a recipient in a case where the donor is present may be the same species or may be species different from each other. In a case where the pancreatic islet is used as insulin-secreting cells, it may be of xenogeneic origin or of allogeneic origin. In a case where cells are used, they are often of allogeneic and heterogeneous origin or of autologous origin; however, they may be of allogeneic origin. In a case where mesenchymal stem cells are used in the cell structure, the mesenchymal stem cells and a recipient may be of allogeneic origin or xenogeneic origin with each other. In a case of being of allogeneic origin, it may be of autologous origin or of heterogeneous origin.

It is preferable that the number of insulin-secreting cells to be used for the treatment of a disease that requires the transplantation of transplanting insulin-secreting cells is small. This is because as the number of cells increases, the volume increases, which not only makes transplantation difficult but also increases foreign body sensation after the transplantation. In a case of using rat-derived pancreatic islets as insulin-secreting cells that are used to normalize the blood glucose level in a diabetic mouse in which diabetes is induced with streptozotocin, it is preferable to use 100 or more and less than 5,000 pancreatic islets, more preferable to use 100 or more and less than 2,500 pancreatic islets, and still more preferable to use 100 or more and less than 1,250 pancreatic islets. It is known that the number of pancreatic islets required to maintain a normal blood glucose level varies greatly between species, and it is said that it roughly depends on body weight. Accordingly, the above numbers of pancreatic islets are mentioned only as a guide.

The present invention will be more specifically described using the following examples; however, it is not limited by the examples.

### Examples

### (1) Preparation of diabetic mouse

Streptozotocin (manufactured by FUJIFILM Wako Pure Chemical Corporation) was suspended in water for injection (Otsuka Pharmaceutical Factory, Inc.), and an amount of 250 mg/kg per mouse was administered intraperitoneally to a 6-week-old Balb/c mouse (Charles River Laboratories Japan, Inc.). One week after administration of streptozotocin, a mouse having a blood glucose level of 300 mg/dL was used as a diabetic-developed mouse as a recipient of cell transplantation.

### (2) Isolation of rat pancreatic islet

A Wistar rat was prepared, and 1 mg/mL Collagenase type V (manufactured by Sigma-Aldrich Co., LLC) was injected through the pancreatic duct to swell the pancreas, and the swollen pancreas was removed. The removed pancreas was digested at 37°C, and then pancreatic islets were purified by density gradient centrifugation at 900 g using 10 mL of Lymphoprep (manufactured by Cosmo Bio Co., Ltd.) and 10 mL of HBSS (FUJIFILM Wako Pure Chemical Corporation) for 20 minutes.

### (3) Preparation of cell structure

Mouse adipose-derived mesenchymal stem cells (mADSC) were suspended in D-MEM medium (Dulbecco's modified Eagle's medium) containing 10% fetal bovine serum (FBS), and a biocompatible polymer block (53 to 106 µm) (the biocompatible polymer block described in Example 2 of JP2015-134193A) was added thereto. In a final state where mADSC (1.2 × 10⁶ cells) and the biocompatible polymer block (0.25 mg) were suspended in 4 ml of the medium, the suspension was seeded in an EZSPHERE (registered trade name) dish Type 903 (spheroid well diameter: 800 µm, spheroid well depth: 300 µm, spheroid well number: about 1,200 wells, the bottom surface is a culture surface having a recessed portion, and has an outer side wall portion erected on the peripheral edge of the culture surface, manufactured by AGC TECHNO GLASS Co., Ltd.), which is a cell non-adhesive 35 mm dish. The dish was allowed to stand in a CO₂ incubator at 37°C and in a 5% CO₂ atmosphere for 48 hours to obtain about 1,000 cell structures (size: about 200 µm) which were uniform.

### (4) Preparation of sodium alginate microencapsulated pancreatic islet (pancreatic islet encapsulated in capsule, without cell structure) (comparative example)

Pancreatic islets isolated from a Wistar rat were suspended in a 3.9% by mass of a sodium alginate solution (KIMICA, low endotoxin sodium alginate AL10) so that the rate of 0.05 to 0.15 µL per pancreatic islet was maintained. The pancreatic islets were homogeneously dispersed by repeatedly moving the entire suspension between two syringes. Using Encapsulator B-395 Pro manufactured by BUCHI Labortechnik AG, the suspension was dropwise added to 100 mmol/L calcium chloride under stirring, whereby sodium alginate microencapsulated pancreatic islets were obtained. Alginate capsules were coated with 0.05% by mass poly-L-ornithine (Sigma-Aldrich Co., LLC) for 6 minutes.

### (5) Intraperitoneal transplantation of sodium alginate microencapsulated pancreatic islet (pancreatic islet encapsulated in capsule, without cell structure) (comparative example)

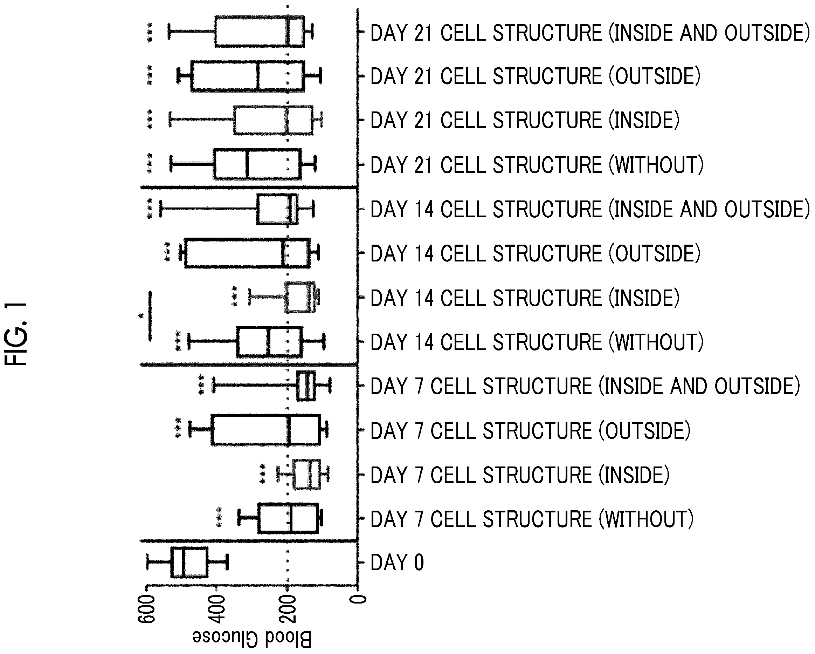

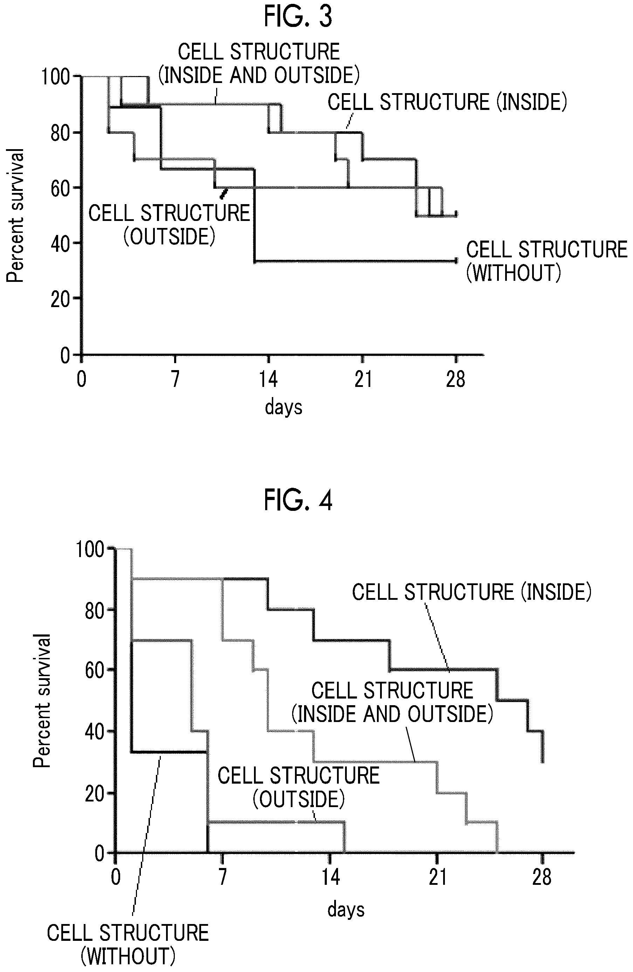

To the diabetic mouse prepared in (1), 500 islets of the sodium alginate microencapsulated pancreatic islet obtained in (4) were suspended in a medium containing 3 mmol/L glucose (Cosmo Bio Co., Ltd.: PNIM4, medium composition; RPMI1640, serum, glucose, and the like) and administered intraperitoneally. The blood glucose level and the body weight of the mouse were observed daily. Fig. 1 shows the blood glucose level. In addition, Fig. 3 shows the control rate of blood glucose level in a case where drop-out is defined as the blood glucose level of 250 mg/dL or more in two consecutive measurements.

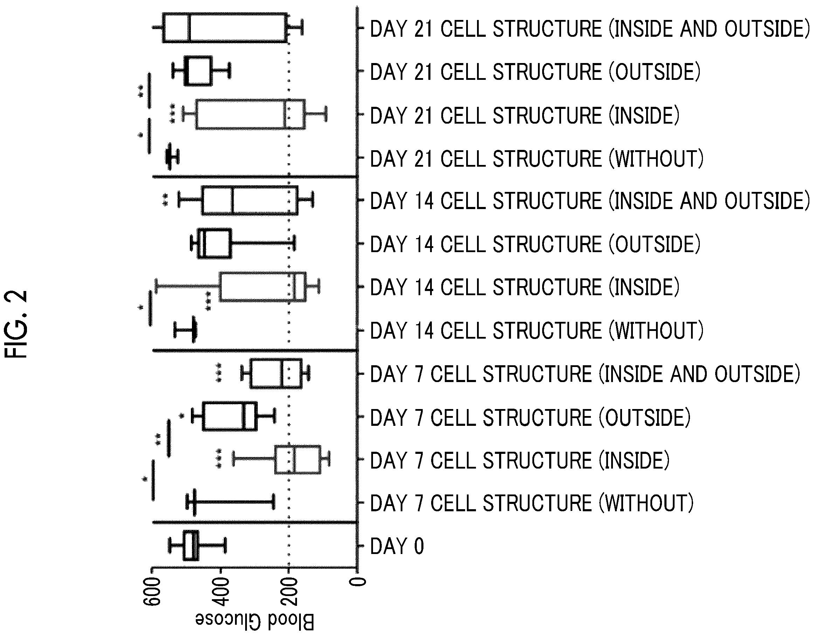

The vertical axis in Fig. 1 and Fig. 2 indicates the blood glucose level (mg/dL).

The vertical axis in Fig. 2 and Fig. 4 indicates the control rate (%) of blood glucose level.

The control rate of blood glucose level was determined as % by dividing the number of mice that did not drop out from the blood glucose level control by the number of all mice. It is noted that the "drop-out from the blood glucose level control" is defined as a drop-out mouse when the blood glucose level exceeded 250 mg/dL consecutively in two blood glucose measurements.

In Fig. 1 to Fig. 4, Cell structure (without) indicates a comparative example of the (pancreatic islet encapsulated in capsule, without cell structure).

In Fig. 1 to Fig. 4, Cell structure (outside) indicates a comparative example of the (pancreatic islet encapsulated in capsule, cell structure arranged outside capsule).

In Fig. 1 to Fig. 4, Cell structure (inside) indicates the present invention of the (pancreatic islet encapsulated in capsule, cell structure arranged inside capsule).

In Fig. 1 to Fig. 4, Cell structure (inside and outside) indicates the present invention of the (pancreatic islet encapsulated in capsule, cell structure arranged both inside capsule and outside capsule).

### (6) Subcutaneous transplantation of sodium alginate microencapsulated pancreatic islet (pancreatic islet encapsulated in capsule, without cell structure) (comparative example)

To the diabetic mouse prepared in (1), 500 islets of the sodium alginate microencapsulated pancreatic islet obtained in (4) were suspended in a medium containing 3 mmol/L glucose (Cosmo Bio Co., Ltd.: PNIM4) and administered subcutaneously. The blood glucose level and the body weight of the mouse were observed daily. Fig. 2 shows the blood glucose level. **In** addition, Fig. 4 shows the control rate of blood glucose level when the blood glucose level of 250 mg/dL or more was dropped in two consecutive measurements.

### (7) Intraperitoneal transplantation of sodium alginate microencapsulated pancreatic islet (pancreatic islet encapsulated in capsule, cell structure arranged outside capsule) (comparative example)

To the diabetic mouse prepared in (1), 500 islets of the sodium alginate microencapsulated pancreatic islet obtained in (4), and the cell structures obtained in (3), which had been suspended in a medium containing 3 mmol/L glucose (Cosmo Bio Co., Ltd.: PNIM4), were simultaneously administered intraperitoneally. The blood glucose level and the body weight of the mouse were observed daily. Fig. 1 shows the blood glucose level. **In** addition, Fig. 3 shows the control rate of blood glucose level in a case where drop-out is defined as the blood glucose level of 250 mg/dL or more in two consecutive measurements.

### (8) Subcutaneous transplantation of sodium alginate microencapsulated pancreatic islet (pancreatic islet encapsulated in capsule, cell structure arranged outside capsule) (comparative example)

To the diabetic mouse prepared in (1), 500 islets of the sodium alginate microencapsulated pancreatic islet obtained in (4) and 500 structures of the cell structure obtained in (3) were suspended in a medium containing 3 mmol/L glucose (Cosmo Bio Co., Ltd.: PNIM4) and administered subcutaneously. The blood glucose level and the body weight of the mouse were observed daily. Fig. 2 shows the blood glucose level. In addition, Fig. 4 shows the control rate of blood glucose level when the blood glucose level of 250 mg/dL or more was dropped in two consecutive measurements.

### (9) Preparation of sodium alginate microencapsulated pancreatic islet (pancreatic islet encapsulated in capsule, cell structure arranged inside capsule) (present invention)

Pancreatic islets isolated from a Wistar rat and the cell structures prepared in (3) were mixed and suspended in a 3.9% by mass of a sodium alginate solution (KIMICA, low endotoxin sodium alginate AL10) so that the rate of 0.05 to 0.15 µL per pancreatic islet and per cell structure was maintained. The pancreatic islets were homogeneously dispersed by repeatedly moving the entire suspension between two syringes. Using Encapsulator B-395 Pro manufactured by BUCHI Labortechnik AG, the above-described suspension was dropwise added to 100 mmol/L calcium chloride under stirring, whereby sodium alginate microencapsulated pancreatic islets were obtained. Alginate capsules were coated with 0.05% by mass poly-L-ornithine (Sigma-Aldrich Co., LLC) for 6 minutes. The average circle-equivalent diameter size of the pancreatic islets was 207.5 µm, the average circle-equivalent diameter size of the cell structures was 199.4 µm, and the circle-equivalent diameter size of the cell structure was 0.96 times that of the pancreatic islet. 1,000 islets of the pancreatic islet and 1,000 structures of the cell structure were measured and average values were calculated. The encapsulation number ratio (pancreatic islet:cell structure) of the cell structures to the pancreatic islets was 1.15:1. The ratio of the cell structures to pancreatic islets was 1:0.87.

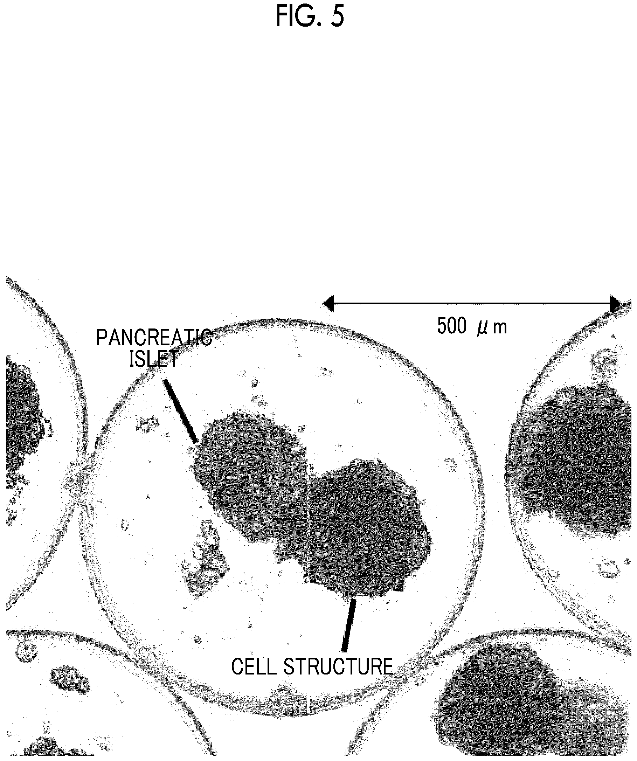

Fig. 5 is an image showing sodium alginate microcapsule that encapsulates pancreatic islets and cell structures.

### (10) Intraperitoneal transplantation of sodium alginate microencapsulated pancreatic islet (pancreatic islet encapsulated in capsule, cell structure arranged inside capsule) (present invention)

To the diabetic mouse prepared in (1), 500 islets of the sodium alginate microencapsulated pancreatic islet obtained in (9) were suspended in a medium containing 3 mmol/L glucose (Cosmo Bio Co., Ltd.: PNIM4) and administered intraperitoneally. The blood glucose level and the body weight of the mouse were observed daily. Fig. 1 shows the blood glucose level. In addition, Fig. 3 shows the control rate of blood glucose level in a case where drop-out is defined as the blood glucose level of 250 mg/dL or more in two consecutive measurements.

### (11) Subcutaneous transplantation of sodium alginate microencapsulated pancreatic islet (pancreatic islet encapsulated in capsule, cell structure arranged inside capsule) (present invention)

To the diabetic mouse prepared in (1), 500 islets of the sodium alginate microencapsulated pancreatic islet obtained in (9) were suspended in a medium containing 3 mmol/L glucose (Cosmo Bio Co., Ltd.: PNIM4) and administered subcutaneously. The blood glucose level and the body weight of the mouse were observed daily. Fig. 2 shows the blood glucose level. In addition, Fig. 4 shows the control rate of blood glucose level when the blood glucose level of 250 mg/dL or more was dropped in two consecutive measurements.

### (12) Intraperitoneal transplantation of sodium alginate microencapsulated pancreatic islet (pancreatic islet encapsulated in capsule, cell structure arranged both inside capsule and outside capsule) (present invention)

To the diabetic mouse prepared in (1), 500 islets of the sodium alginate microencapsulated pancreatic islet obtained in (9) and 500 structures of the cell structure obtained in (3) were suspended in a medium containing 3 mmol/L glucose (Cosmo Bio Co., Ltd.: PNIM4) and administered intraperitoneally. The blood glucose level and the body weight of the mouse were observed daily. Fig. 1 shows the blood glucose level. In addition, Fig. 3 shows the control rate of blood glucose level in a case where drop-out is defined as the blood glucose level of 250 mg/dL or more in two consecutive measurements.

### (13) Subcutaneous transplantation of sodium alginate microencapsulated pancreatic islet (pancreatic islet encapsulated in capsule, cell structure arranged both inside capsule and outside capsule) (present invention)

To the diabetic mouse prepared in (1), 500 islets of the sodium alginate microencapsulated pancreatic islet obtained in (9) and 500 structures of the cell structure obtained in (3) were suspended in a medium containing 3 mmol/L glucose (Cosmo Bio Co., Ltd.: PNIM4) and administered subcutaneously. The blood glucose level and the body weight of the mouse were observed daily. Fig. 2 shows the blood glucose level. In addition, Fig. 4 shows the control rate of blood glucose level when the blood glucose level of 250 mg/dL or more was dropped in two consecutive measurements.

### (14) Summary of transplantation result

From the blood glucose levels in the intraperitoneal transplantation shown in Fig. 1, good blood glucose level control was observed since in the group in which no cell structure was contained and the group in which the cell structure was arranged outside the capsule, the number of mice of which blood glucose level was less than 200 mg/dL on the 7th day of transplantation was 50%, whereas in the group in which the cell structure was arranged inside the capsule and in the group in which the cell structure was arranged inside and outside the capsule, the blood glucose level was less than 200 mg/dL in 75% or more of the mice. In the group in which the cell structure was arranged inside the capsule, 75% of the mice had a blood glucose level of 200 mg/dL or less even on the 14th day of transplantation, which indicated the significantly best blood glucose level control. It was found that in the group in which the cell structure was arranged inside the capsule and in the group in which the cell structure was arranged inside and outside the capsule, 50% of the mice achieved a blood glucose level of 200 mg/dL or less even on the 21st day of transplantation, which was a good result as compared with other groups.

From the blood glucose levels in the subcutaneous transplantation shown in Fig. 2, significantly good blood glucose level control was observed since in the group in which no cell structure was contained and the group in which the cell structure was arranged outside the capsule, 0% of the mice achieved a blood glucose level of 200 mg/dL or less on the 7th day of transplantation, whereas in the group in which the cell structure was arranged inside the capsule and in the group in which the cell structure was arranged inside and outside the capsule, about 50% of the mice achieved a blood glucose level of 200 mg/dL. It was observed that in the group in which the cell structure was arranged inside the capsule and in the group in which the cell structure was arranged inside and outside the capsule, about 50% and about 25% of the mice achieved a blood glucose level of 200 mg/dL or less even on the 14th day of transplantation, and thus significantly good blood glucose level control was observed. Similarly, it was observed that in the group in which the cell structure was arranged inside the capsule and in the group in which the cell structure was arranged inside and outside the capsule, about 50% and about 25% of the mice achieved a blood glucose level of 200 mg/dL or less even on the 21st day of transplantation, and thus significantly good blood glucose level control was observed. In addition, it was found that the group in which the cell structure was arranged in the capsule exhibits the best blood glucose level control performance at any time point.

From the changes in the control rate of blood glucose level in Fig. 3 and Fig. 4, it was found that the group in which the cell structure was arranged in the capsule shows the best result in any transplantation system of the intraperitoneal transplantation or the subcutaneous transplantation. Regarding the order of the results, the best is the group in which the cell structure is arranged inside the capsule, the next is the group in which the cell structure is arranged inside and outside the capsule, which is followed by the group in which the cell structure is arranged outside the capsule, and the worst is the group in which no cell structure is contained. In addition, as shown in Fig. 4, the difference in result became clearer in the subcutaneous transplantation system, which is a severe system.

From these results, it was revealed that the inclusion of the cell structure provides a high treatment effect and that in a case where the cell structure is in a state of being encapsulated in the capsule, a remarkably high treatment effect is further exhibited.

### (15) Preparation of sodium alginate microencapsulated cell structure

The cell structures prepared in (3) were mixed and suspended in a 3.9% of a concentration of a sodium alginate solution (KIMICA, low endotoxin sodium alginate AL10) so that the rate of 0.05 to 0.15 µL per cell structure was maintained. The cell structures were homogeneously dispersed by repeatedly moving the entire suspension between two syringes. Using Encapsulator B-395 Pro manufactured by BUCHI Labortechnik AG, the above-described suspension was dropwise added to 100 mmol/L calcium chloride under stirring, whereby sodium alginate microencapsulated cell structures were obtained. Alginate capsules were coated with 0.05% by mass poly-L-ornithine (Sigma-Aldrich Co., LLC) for 6 minutes.
[Sequence Listing] International application application based on the International Patent Cooperation Treaty 19F01518W1JP20031546_0.app

## Claims

1. A composition comprising at least:
(a) a microcapsule containing a polymer hydrogel;
(b) a cell structure containing a biocompatible polymer block and a cell, in which a plurality of biocompatible polymer blocks are arranged in gaps between a plurality of cells; and
(c) one or more selected from an insulin-secreting cell, a pancreatic islet cell, and a pancreatic islet,
wherein at least a part of the cell structures and at least a part of the one or more selected from an insulin-secreting cell, a pancreatic islet cell, or a pancreatic islet are encapsulated in the microcapsules,
wherein the cell is a somatic stem cell, and
wherein the biocompatible polymer blocks are constituted by a polymer composed of at least one biodegradable material selected from the group consisting of a polypeptide, a polylactic acid, a polyglycolic acid, polylactic acid-co-glycolic acid (PLGA), hyaluronic acid, glycosaminoglycan, proteoglycan, chondroitin, cellulose, agarose, carboxymethyl cellulose, chitin, and chitosan.

2. The composition according to claim 1,
wherein the cell is a mesenchymal stem cell.

3. The composition according to claims 1 or 2,
wherein at least a part of the cell structures are present outside the microcapsules.

4. The composition according to any one of claims 1 to 3,
wherein 50% or more of the cell structures are present inside the microcapsules.

5. The composition according to any one of claims 1 to 4,
wherein 50% or more of the (c) one or more selected from an insulin-secreting cell, a pancreatic islet cell, or a pancreatic islet are encapsulated in the microcapsules.

6. The composition according to any one of claims 1 to 5,
wherein the composition contains a pancreatic islet, and
a circle-equivalent diameter size of the cell structure is 0.5 times or more and 4 times or less with respect to the pancreatic islet.

7. The composition according to any one of claims 1 to 6,
wherein the composition contains a pancreatic islet, and
a number ratio of the cell structures to the pancreatic islets, which are encapsulated in one microcapsule, is in a range of 0.1:1.0 to 1.0:0.1.

8. The composition according to any one of claims 1 to 7,
wherein a main component of the microcapsule is alginic acid.

9. The composition according to any one of claims 1 to 8,
wherein the microcapsule is coated with poly-L-ornithine.

10. The composition according to any one of claims 1 to 9,
wherein the composition is used for transplantation into a living body.

11. The composition according to any one of claims 1 to 10,
wherein the composition is for intraperitoneal transplantation or subcutaneous transplantation.

12. The composition according to claim 11,
wherein the composition is for subcutaneous transplantation.

13. The composition according to any one of claims 1 to 12,
wherein the composition is a pharmaceutical composition.

14. The composition according to any one of claims 1 to 13,
wherein the composition is a pharmaceutical composition for treatment of diabetes.

## Patentansprüche

1. Zusammensetzung, die mindestens umfasst:
(a) eine Mikrokapsel, die ein Polymerhydrogel enthält;
(b) eine Zellstruktur, die einen biokompatiblen Polymerblock und eine Zelle enthält, wobei mehrere biokompatible Polymerblöcke in Zwischenräumen zwischen mehreren Zellen angeordnet sind; und
(c) eine oder mehrere, die aus einer Insulin-sezernierenden Zelle, einer Langerhans-Inselzelle und einer Langerhans-Insel ausgewählt sind,
wobei mindestens ein Teil der Zellstrukturen und mindestens ein Teil der einen oder mehreren, die aus einer Insulin-sezernierenden Zelle, einer Langerhans-Inselzelle oder einer Langerhans-Insel ausgewählt sind, in den Mikrokapseln gekapselt sind, wobei die Zelle eine somatische Stammzelle ist, und
wobei die biokompatiblen Polymerblöcke durch ein Polymer, das aus mindestens einem biologisch abbaubaren Material, das aus der Gruppe, die aus einem Polypeptid, einer Polymilchsäure, einer Polyglykolsäure, Polymilchsäure-Co-Glykolsäure (polylactic acid-co-glycolic acid, PLGA), Hyaluronsäure, Glykosaminoglykan, Proteoglykan, Chondroitin, Zellulose, Agarose, Carboxymethylcellulose, Chitin und Chitosan besteht, ausgewählt ist, besteht, aufgebaut sind.

2. Zusammensetzung nach Anspruch 1,
wobei die Zelle eine mesenchymale Stammzelle ist.

3. Zusammensetzung nach Anspruch 1 oder 2,
wobei mindestens ein Teil der Zellstrukturen außerhalb der Mikrokapseln vorhanden ist.

4. Zusammensetzung nach einem der Ansprüche 1 bis 3,
wobei 50 % oder mehr der Zellstrukturen innerhalb der Mikrokapseln vorhanden sind.

5. Zusammensetzung nach einem der Ansprüche 1 bis 4,
wobei 50 % oder mehr der (c) eine oder mehrere, die aus einer Insulin-sezernierenden Zelle, einer Langerhans-Inselzelle oder einer Langerhans-Insel ausgewählt sind, in den Mikrokapseln gekapselt sind.

6. Zusammensetzung nach einem der Ansprüche 1 bis 5,
wobei die Zusammensetzung eine Langerhans-Insel enthält, und
eine kreisäquivalente Durchmessergröße der Zellstruktur 0,5 -mal oder mehr und 4-mal oder weniger in Bezug auf die Langerhans-Insel beträgt.

7. Zusammensetzung nach einem der Ansprüche 1 bis 6,
wobei die Zusammensetzung eine Langerhans-Insel enthält, und
ein Zahlenverhältnis der Zellstrukturen zu den Langerhans-Inseln, die in einer Mikrokapsel gekapselt sind, in einem Bereich von 0,1:1,0 bis 1,0:0,1 liegt.

8. Zusammensetzung nach einem der Ansprüche 1 bis 7,
wobei eine Hauptkomponente der Mikrokapsel Alginsäure ist.

9. Zusammensetzung nach einem der Ansprüche 1 bis 8,
wobei die Mikrokapsel mit Poly-L-Ornithin beschichtet ist.

10. Zusammensetzung nach einem der Ansprüche 1 bis 9,
wobei die Zusammensetzung zur Transplantation in einen lebenden Körper verwendet wird.

11. Zusammensetzung nach einem der Ansprüche 1 bis 10,
wobei die Zusammensetzung für intraperitoneale Transplantation oder subkutane Transplantation ist.

12. Zusammensetzung nach Anspruch 11,
wobei die Zusammensetzung für subkutane Transplantation ist.

13. Zusammensetzung nach einem der Ansprüche 1 bis 12,
wobei die Zusammensetzung eine pharmazeutische Zusammensetzung ist.

14. Zusammensetzung nach einem der Ansprüche 1 bis 13,
wobei die Zusammensetzung eine pharmazeutische Zusammensetzung zur Behandlung von Diabetes ist.

## Revendications

1. Composition comprenant au moins :
(a) une microcapsule contenant un hydrogel de polymère ;
(b) une structure cellulaire contenant un bloc de polymère biocompatible et une cellule, dans laquelle une pluralité de blocs de polymère biocompatible sont disposés dans des espaces entre une pluralité de cellules ; et
(c) un ou plusieurs sélectionnés parmi une cellule sécrétant de l'insuline, une cellule d'îlot pancréatique et un îlot pancréatique,
dans laquelle au moins une partie des structures cellulaires et au moins une partie d'un ou plusieurs sélectionnés parmi une cellule sécrétant de l'insuline, une cellule d'îlot pancréatique ou un îlot pancréatique sont encapsulés dans les microcapsules,
dans laquelle la cellule est une cellule souche somatique, et
dans laquelle les blocs de polymère biocompatible sont constitués d'un polymère composé d'au moins un matériau biodégradable sélectionné parmi le groupe constitué d'un polypeptide, d'un acide polylactique, d'un acide polyglycolique, d'acide polylactique-co-glycolique (PLGA), d'acide hyaluronique, de glycosaminoglycane, de protéoglycane, de chondroïtine, de cellulose, d'agarose, de carboxyméthylcellulose, de chitine et de chitosane.

2. Composition selon la revendication 1,
dans laquelle la cellule est une cellule souche mésenchymateuse.

3. Composition selon la revendication 1 ou la revendication 2,
dans laquelle au moins une partie des structures cellulaires est présente à l'extérieur des microcapsules.

4. Composition selon l'une quelconque des revendications 1 à 3,
dans laquelle 50 % ou plus des structures cellulaires sont présentes à l'intérieur des microcapsules.

5. Composition selon l'une quelconque des revendications 1 à 4,
dans laquelle 50 % ou plus de (c) un ou plusieurs sélectionnés parmi une cellule sécrétant de l'insuline, une cellule d'îlot pancréatique ou un îlot pancréatique sont encapsulés dans les microcapsules.

6. Composition selon l'une quelconque des revendications 1 à 5,
dans laquelle la composition contient un îlot pancréatique, et
une taille de diamètre équivalent en cercle de la structure cellulaire est 0,5 fois ou plus et 4 fois ou moins par rapport à l'îlot pancréatique.

7. Composition selon l'une quelconque des revendications 1 à 6,
dans laquelle la composition contient un îlot pancréatique, et
un rapport de nombre des structures cellulaires aux îlots pancréatiques, qui sont encapsulés dans une microcapsule, est dans une plage de 0,1:1,0 à 1,0:0,1.

8. Composition selon l'une quelconque des revendications 1 à 7,
dans laquelle un composant principal de la microcapsule est de l'acide alginique.

9. Composition selon l'une quelconque des revendications 1 à 8,
dans laquelle la microcapsule est revêtue de poly-L-ornithine.

10. Composition selon l'une quelconque des revendications 1 à 9,
dans laquelle la composition est utilisée pour une transplantation dans un corps vivant.

11. Composition selon l'une quelconque des revendications 1 à 10,
dans laquelle la composition est destinée à une transplantation intrapéritonéale ou à une transplantation sous-cutanée.

12. Composition selon la revendication 11,
dans laquelle la composition est destinée à une transplantation sous-cutanée.

13. Composition selon l'une quelconque des revendications 1 à 12,
dans laquelle la composition est une composition pharmaceutique.

14. Composition selon l'une quelconque des revendications 1 à 13,
dans laquelle la composition est une composition pharmaceutique pour le traitement du diabète.
